# EUROPÄISCHE PATENTANMELDUNG

(11) **EP 3 167 860 A1**
(43) Veröffentlichungstag der Anmeldung: **17.05.2017**
(21) Anmeldenummer: 16194218.0
(22) Anmeldetag: 17.10.2016
(51) Int. Cl.: A61F 13/551, A61F 13/514, B32B 7/06, B32B 7/12, B32B 25/08, B32B 25/16, B32B 27/08, B32B 27/20, B32B 27/30, B32B 27/32, A61F 15/00

(54) **MEHRLAGIGE EINSCHLAGFOLIE FÜR HYGIENEPRODUKTE, INSBESONDERE DAMENBINDEN**

(30) Priorität: 16.11.2015 US 201514941761
(71) Anmelder: Mondi Consumer Packaging Technologies GmbH, 48599 Gronau (DE)
(72) Erfinder: Bader, Herbert, 92442 Wackersdorf (DE); Stoltz, Timothy, Dr West Chester, OH 45069 (US); Niepelt, Ralf, 48599 Gronau (DE)
(74) Vertreter: Lorenz, Bernd Ingo Thaddeus

(57) **Zusammenfassung**

Die Erfindung betrifft eine mehrlagige Einschlagfolie (2) für Hygieneprodukte, insbesondere Damenbinden (1), mit einer ersten Außenlage (6), einer zweiten Außenlage (7) und mit einer Haftklebstoffschicht zwischen der ersten Außenlage (6) und der zweiten Außenlage (7), wobei die Verbundhaftung zwischen der Haftklebstoffschicht und der ersten Außenlage (6) größer ist als die Verbundhaftung zwischen der Haftklebstoffschicht und der zweiten Außenlage (7) und wobei die Gesamtdicke der ersten und zweiten Außenlage (6, 7) mit der dazwischen angeordneten Haftklebstoffschicht zwischen 20 µm und 80 µm liegt.

## Beschreibung

Die Erfindung betrifft eine mehrlagige Einschlagfolie für Hygieneprodukte, insbesondere Damenbinden. Gegenstand der Erfindung ist des Weiteren eine einzeln eingeschlagene Damenbinde.

Die Einschlagfolie erstreckt sich bezogen auf einen flachgelegten Zustand über die Damenbinde hinaus. Bei der Herstellung werden dann in einer Längsrichtung der Damenbinde gesehen die Enden nacheinander umgefaltet, so dass sich eine dreilagige Ausgestaltung ergibt, bei der die Enden der Damenbinde jeweils von der Einschlagfolie geschützt auf einen mittleren Abschnitt der Damenbinde umgelegt sind. Die Fixierung kann durch seitliche Prägungen und Siegelungen und/oder einen Klebestreifen erfolgen.

Entsprechende Einschlagfolien und damit einzeln eingeschlagene Damenbinden sind aus EP 0 532 649 B2 und EP 0 639 147 B2 bekannt.

Ausgehend von der im Stand der Technik beschriebenen Ausgestaltung werden in der Praxis Einschlagfolien verwendet, welche eine Gesamtdicke von lediglich 16 µm aufweisen, dreischichtig coextrudiert sind und an ihrer der Damenbinde zugewandten Seite mit einer Silikon-Beschichtung versehen sind. Üblicherweise sind die bekannten Einschlagfolien auch eingefärbt, wozu ein Weißbatch oder ein anderer Farbbatch vorgesehen sein können. Darüber hinaus können die Folien auch bedruckt, geprägt oder mit Einschnitten versehen sein.

Gerade eine sehr dünne Folie kann von einem Benutzer als geringwertig angesehen werden, wobei diesem Eindruck durch eine Prägung in einem gewissen Maße entgegengewirkt werden kann. Dieses Problem ist besonders eminent, da zum Zwecke der Materialersparnis noch geringere Foliendicken von beispielsweise 13 µm bis 14 µm angestrebt werden.

Um die einzeln eingeschlagenen Damenbinden bereitzustellen, wird eine Bahn der Einschlagfolie zugeführt und auf der mit der Silikon-Beschichtung versehenen Seite mit einem heißschmelzenden Haftklebstoff versehen, der beispielsweise bei einer Temperatur von 160 °C bis 180 °C aufgebracht wird. Auch diese Temperaturbelastung kann gerade bei sehr dünnen Folien zu einer unerwünschten Verformung führen.

Der Auftrag des Haftklebstoffes erfolgt lediglich bereichsweise, wobei dann einzelne Damenbinden über dem Haftklebstoff appliziert werden. Durch die Silikon-Beschichtung wird sichergestellt, dass nachfolgend der Haftklebstoff an der Unterseite der Damenbinde verbleibt, und sich zuverlässig von der Einschlagfolie trennen kann.

Zweckmäßigerweise werden die einzelnen Damenbinden quer zu einer Produktionsrichtung aufgelegt, wobei dann die Ränder der Einschlagfolie mit den darauf abgelegten Damenbinden umgeklappt werden, um die bekannte, C-förmig gefaltete Form der einzeln eingeschlagenen Damenbinden zu erreichen. Die Fixierung kann dann sowohl durch ein Prägen zwischen den aufeinanderfolgenden Damenbinden und die Anbringung eines Klebestreifens erfolgen, wobei bevorzugt eine Kombination dieser beiden Maßnahmen vorgesehen ist. Abschließend wird der so gebildete Schlauch in Stücke mit jeweils einer Damenbinde geschnitten.

Neben den bereits genannten Nachteilen ist zu berücksichtigen, dass sowohl bei der Herstellung als auch später bei der Benutzung aufgrund der geringen Gesamtdicke der Einschlagfolie die Gefahr eines Zerreißens besteht.

Darüber hinaus erfordert die Anordnung des Haftklebstoffes in einem genau vorgegebenen Muster und die lagerichtige Anordnung der einzelnen Damenbinden darauf eine sorgfältige Verfahrensführung sowie einen hohen apparativen Aufwand.

Der Begriff Damenbinde umfasst im Rahmen der vorliegenden Erfindung auch Slipeinlagen, Inkontinenzprodukte und andere flächige absorbierende Hygieneprodukte, die einzeln mit einer Einschlagfolie verpackt werden.

Der vorliegenden Erfindung liegt die Aufgabe zugrunde, die aus dem Stand der Technik bekannten Nachteile zu vermeiden. Insbesondere soll vermieden werden, dass unmittelbar bei der Herstellung der einzeln eingeschlagenen Damenbinde Haftklebstoff im schmelzflüssigen Zustand aufgebracht werden muss.

Dabei ist auch zu berücksichtigen, dass die Einschlagfolie als Vorprodukt zugeliefert und weiterverarbeitet wird. Im Rahmen der Erfindung soll gerade die Weiterverarbeitung erleichtert werden.

Gegenstand der Erfindung und Lösung der Aufgabe ist eine mehrlagige Einschlagfolie für Hygieneprodukte, insbesondere Damenbinden, mit einer ersten Außenlage, einer zweiten Außenlage und mit einer Haftklebstoffschicht zwischen der ersten Außenlage und der zweiten Außenlage, wobei die Verbundhaftung zwischen der Haftklebstoffschicht und der ersten Außenlage größer ist als die Verbundhaftung zwischen der Haftklebstoffschicht und der zweiten Außenlage und wobei die Gesamtdicke der ersten und zweiten Außenlage mit der dazwischen angeordneten Haftklebstoffschicht zwischen 20 µm und 80 µm liegt.

Erfindungsgemäß befindet sich der Haftklebstoff, mit dem die Damenbinde nachfolgend bei ihrem Gebrauch in dem Schrittbereich einer Unterhose angeordnet wird, zunächst innerhalb der mehrlagigen Einschlagfolie, wobei durch eine feste Verklebung der ersten Außenlage mit zumindest einem Grundkörper der Damenbinde die mehrlagige Einschlagfolie lokal aufgerissen wird, wodurch die erste Außenlage mit der Haftklebstoffschicht an der Unterseite der Damenbinde verbleibt.

Gegenstand der Erfindung ist entsprechend auch eine einzeln eingeschlagene Damenbinde mit einem sich über die Damenbinde hinauserstreckenden Zuschnitt der mehrlagigen Einschlagfolie, wobei zumindest ein Grundkörper der Damenbinde nicht lösbar mit der ersten Außenlage verklebt ist. Unter nicht lösbar wird im Rahmen der vorliegenden Erfindung verstanden, wenn sich die Verklebung zwischen der Damenbinde oder zumindest einem Grundkörper der Damenbinde bei dem Abziehen der Damenbinde von der Einschlagfolie nicht von der ersten Außenlage trennen kann und vielmehr die erste Außenlage am Rand der nicht lösbaren Verklebung mit dem darunter angeordneten Haftklebstoff aufbricht und sich dann flächig von der zweiten Außenlage trennt.

Zur nicht lösbaren Verklebung können verschiedene chemisch und/oder physikalisch aushärtende Klebstoffe vorgesehen sein. Neben lösungsmittelhaltigen Klebstoffen und Mehrkomponentenklebstoffen kommen grundsätzlich auch Heißklebstoffe mit einer ausreichend hohen Festigkeit in Betracht.

In den nicht lösbar zu verklebenden Abschnitten der ersten Außenlage mit der Damenbinde bzw. einem Grundkörper der Damenbinde kann der Klebstoff sowohl flächig als auch in einem Muster aufgebracht werden. Bei einem Klebstoffmuster kann beispielsweise auch ein umlaufender Rahmen vorgesehen sein, um einen sicheren Halt an den am meistbelasteten Stellen zu gewährleisten.

Im Rahmen der Erfindung ergeben sich mehrere Vorteile. Zunächst kann eine insgesamt größere Gesamtdicke der Einschlagfolie zwischen 20 µm und 80 µm auch aus Kostengründen in Kauf genommen werden, weil der Haftklebstoff für die Damenbinde bereits in die Einschlagfolie als Vorprodukt integriert ist. Durch die Gesamtdicke zwischen 20 µm und 80 µm ergibt sich aus Sicht eines Verbrauchers ein höherer Qualitätseindruck.

Darüber hinaus kann im Rahmen der Erfindung an den Außenseiten der mehrlagigen Einschlagfolie vollständig auf eine Silikon-Beschichtung oder eine andere Release-Beschichtung verzichtet werden, so dass die Einschlagfolie auch bei einer geeigneten Materialauswahl leicht heißgesiegelt werden kann. Beispielsweise kann nach einer C-förmigen Faltung eine Fixierung durch Heißsiegelpunkte oder -nähte erfolgen, so dass auf die Anbringung eines separaten Klebestreifens als Verschluss verzichtet werden kann.

Ein weiterer Vorteil ist darin zu sehen, dass die Aufbringung eines einfachen nicht lösbaren Klebstoffes leichter durchgeführt werden kann, als eine Beschichtung mit einem schmelzflüssigen Haftklebstoff.

Im Rahmen der Erfindung muss gewährleistet werden, dass die Verbundhaftung zwischen der Haftklebstoffschicht und der ersten Außenlage größer ist als die Verbundhaftung zwischen der Haftklebstoffschicht und der zweiten Außenlage, damit sich bei einem Abziehen der Damenbinde von der Einschlagfolie der Haftklebstoff von der zweiten Außenlage trennt und gemeinsam mit dem entsprechenden Abschnitt der ersten Außenlage an der Unterseite der Damenbinde verbleibt. Zu diesem Zweck kann die zweite Außenlage eine an die Haftklebstoffschicht angrenzende Release-Schicht aufweisen. Zusätzlich oder alternativ kann zwischen der ersten Außenlage und der Haftklebstoffschicht eine angepasste Haftvermittlerschicht vorgesehen sein, um die Verbundhaftung zu erhöhen.

Grundsätzlich können die erste Außenlage und die zweite Außenlage jeweils aus nur einer oder auch mehreren Folienschichten gebildet sein.

Erfindungsgemäß wird eine mehrlagige Einschlagfolie bereitgestellt, welche zwischen den beiden Außenlagen geschützt die Haftklebstoffschicht aufweist. Die Einschlagfolie kann dabei auf unterschiedliche Weise hergestellt werden.

Gemäß einer ersten Variante sind die beiden Außenlagen und die Haftklebstoffschicht gemeinsam coextrudiert, so dass sich eine besonders einfache Verfahrensführung ergibt. Ein nachträgliches Zusammenführen verschiedener Schichten oder die Aufbringung einer Beschichtung ist im Unterschied zum Stand der Technik dann nicht notwendig.

Alternativ kann die Haftklebstoffschicht auch aus einem thermoplastischen Kunststoff gebildet sein, wobei die erste Außenlage und die zweite Außenlage getrennt gefertigt und durch die Haftklebstoffschicht als Heißklebstoff kaschiert sind. Die Haftklebstoffschicht erfüllt dann zwei unterschiedliche Aufgaben. Einerseits dient die Haftklebstoffschicht im schmelzflüssigen Zustand bei der Herstellung zur Verbindung der ersten Außenlage mit der zweiten Außenlage in einem Kaschierprozess, wobei die Haftklebstoffschicht jedoch dauerhaft klebende Eigenschaften beibehält und in der beschriebenen Weise bei der Benutzung eine Befestigung der Damenbinde an dem Schrittbereich einer Unterhose ermöglicht.

Die Haftklebstoffschicht kann vollflächig zwischen der ersten Außenlage und der zweiten Außenlage vorgesehen sein. Darüber hinaus ist es aber auch möglich, den Haftklebstoff nur in einem Muster oder in Abschnitten vorzusehen, wobei eine solche nicht vollflächige Auftragung insbesondere bei einem Kaschierprozess leicht möglich ist. Ein gleichmäßiges Muster mit Punkten oder einem Gitter kann dazu vorgesehen sein, um insgesamt Haftklebstoff einzusparen, wobei bei einem gleichmäßigen Muster bei der Aufbringung der Damenbinden auf die Einschlagfolie kein vorgegebener Rapport, das heißt keine genaue Positionierung, eingehalten werden muss. Grundsätzlich ist es aber auch möglich, den Haftklebstoff nur in Abschnitten vorzusehen, wobei diese Abschnitte dann später die Befestigungsbereiche für die Damenbinde bilden.

Sofern eine nicht vollflächige Anordnung im Rahmen einer Coextrusion vorgesehen ist, sind gegebenenfalls die Zwischenräume mit einem nicht klebrigen Material aufzufüllen.

Der Haftklebstoff kann beispielsweise auf der Basis von Styrol-Block-Copolymer, insbesondere Styrol-Isopren-Block-Copolymer (SIS) gebildet sein. Styrol-Block-Copolymere sind im besonderen Maße für eine Coextrusion der gesamten mehrlagigen Einschlagfolie geeignet.

Für die Kaschierung mit dem Haftklebstoff als Heißkleber sind insbesondere Acrylat-Klebstoffe geeignet.

Im Rahmen der Erfindung ist nicht nur die Verbundhaftung zwischen den einzelnen Schichten von Bedeutung. Vielmehr muss auch sichergestellt werden, dass an den Rändern der dauerhaften Verklebung der Damenbinde bzw. dem Grundkörper der Damenbinde mit der ersten Außenlage die erste Außenlage der mehrlagigen Einschlagfolie aufbricht. Zu diesem Zweck ist es möglich, die erste Außenlage durch eine geeignete Auswahl der Polymerkomponente und/ oder das Hinzufügen von Zusatzstoffen vergleichsweise brüchig und spröde auszuführen, damit ein Aufbrechen leicht möglich ist.

Zusätzlich oder alternativ kann die erste Außenlage an den Rändern der nicht lösbar verklebten Fläche auch eine Schwächungslinie aufweisen, welche beispielsweise durch einen Laser oder durch ein Stanzen gebildet sein kann. Im Rahmen einer solchen Ausgestaltung muss dann jedoch die Damenbinde bzw. ein Grundkörper der Damenbinde lagerichtig angeordnet werden.

Um geringe Herstellungskosten und gute Funktionseigenschaften zu ermöglichen, weisen die erste Außenlage und die zweite Außenlage gemäß einer bevorzugten Ausgestaltung der Erfindung jeweils als polymeren Hauptbestandteil ein Polyolefin, insbesondere Polyethylen (PE), auf. Im Rahmen einer solchen Ausgestaltung kann dann nach einem Falten der Einschlagfolie ein Verschluss bzw. eine Fixierung durch ein Heißsiegeln erfolgen.

Die Dicke der ersten Außenlage und der zweiten Außenlage liegt typischerweise zwischen 5 µm und 30 µm, wobei die Dicke der Haftklebstoffschicht typischerweise zwischen 5 µm und 25 µm liegt.

Die Haftklebstoffschicht ist nach einem Abziehen der Damenbinde von der Einschlagfolie über die erste Außenlage an der Damenbinde bzw. an dem Grundkörper der Damenbinde befestigt. Vor diesem Hintergrund kann auch vorgesehen sein, dass die erste Außenlage eine Funktionsschicht der gesamten Damenbinde, beispielsweise eine Rückwand der Damenbinde bildet.

Im Rahmen einer solchen Ausgestaltung wird zunächst lediglich ein Grundkörper der Damenbinde bereitgestellt und auf die erste Außenlage aufgebracht und dabei vorzugsweise vollflächig verklebt. Die erste Außenlage bildet dann nach dem Abziehen der Damenbinde von der Einschlagfolie eine Rückwand der Damenbinde. Diese Rückwand kann zur Bereitstellung von atmungsaktiven Eigenschaften auch Poren aufweisen.

Um atmungsaktive Poren der ersten Außenlage zu erzielen, kann zumindest die erste Außenlage gereckt sein. Dabei kann die erste Außenlage Füllstoffe enthalten, wobei an den Füllstoff-Partikeln durch das Recken atmungsaktive Poren gebildet werden. Wenn die gesamte mehrschichtige Einschlagfolie durch das Kaschieren der ersten Außenlage und der zweiten Außenlage gebildet wird, ist ein Recken alleine der ersten Außenlage vor der Kaschierung ausreichend.

Wenn die mehrlagige Einschlagfolie jedoch als Extrusionsfolie hergestellt wird, muss die gesamte Coextrusionsfolie in der beschriebenen Weise gereckt werden, um an Füllstoff-Partikeln der ersten Außenlage atmungsaktive Poren bilden zu können.

Grundsätzlich können Füllstoffe auch vorgesehen sein, um die Einschlagfolie einzufärben und/oder das Aufreißverhalten, insbesondere das Aufbrechen der ersten Außenlage an der nicht lösbaren Verklebung mit der Damenbinde bzw. einem Grundkörper der Damenbinde zu optimieren.

Gemäß einer alternativen Ausgestaltung der Erfindung wird die Damenbinde bereits vollständig mit einer Rückwand bereitgestellt, wobei dann von der in der Einschlagfolie integrierten Haftklebstoffschicht lediglich die klebenden Eigenschaften bereitgestellt werden müssen. Im Rahmen einer solchen Ausgestaltung ist der Grundkörper der Damenbinde mit seiner Rückwand nicht lösbar mit der Außenlage verklebt, wobei die nicht lösbar verklebte Fläche zwischen 20 % und 80 % der Grundfläche der Damenbinde im flachgelegten Zustand beträgt.

Insbesondere können auch mehrere Abschnitte in der beschriebenen Weise nicht lösbar verklebt werden. Besonders bevorzugt sind die Rückwand und die erste Außenlage entlang von zwei vorzugsweise parallel zueinander in einer Längsrichtung der Damenbinde verlaufenden Streifen untrennbar verklebt. Die Längsrichtung der Damenbinde entspricht dabei der Erstreckung eines Schrittbereiches an einer Unterhose.

Die Erfindung wird im Folgenden anhand einer lediglich ein Ausführungsbeispiel darstellenden Zeichnung erläutert. Es zeigen:
- Fig. 1: eine Damenbinde auf einem Abschnitt einer Einschlagfolie im flachliegenden Zustand,
- Fig. 2: eine einzeln in eine Einschlagfolie eingeschlagene Damenbinde,
- Fig. 3A: einen Längsschnitt entlang der Linie A-A der Fig. 1,
- Fig.3B: die Anordnung gemäß der Fig. 3A nach einem Abziehen der Damenbinde von der Einschlagfolie,
- Fig. 4A: die Anordnung gemäß der Fig. 3A in einer alternativen Ausgestaltung,
- Fig. 4B: die Anordnung gemäß der Fig. 4A mit der von der Einschlagfolie abgezogenen Damenbinde.

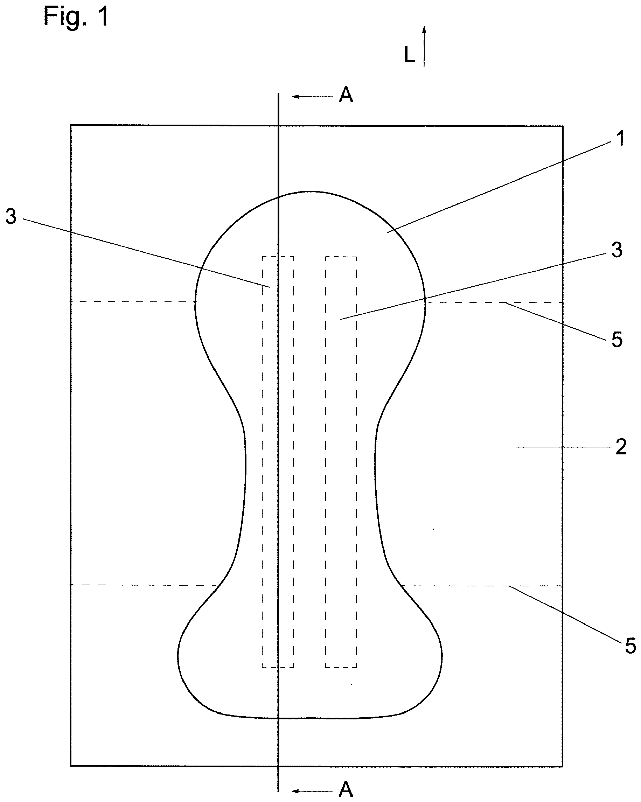

Die Fig. 1 zeigt in einer Draufsicht in einem flachliegenden Zustand eine Damenbinde 1, welche auf einer Einschlagfolie 2 aufliegt.

Um die Damenbinde 1 im Schrittbereich einer Unterhose befestigen zu können, weist diese an ihrer Unterseite Klebebereiche 3 mit einem Haftklebstoff 4 auf, wobei die Klebebereiche 3 in der Fig. 1 angedeutet sind. Die Klebebereiche 3 verlaufen als Streifen parallel zueinander entlang einer Längsrichtung L der Damenbinde 1.

Die Damenbinde 1 kann in einem mittleren Abschnitt auch in nicht dargestellter Weise mit seitlichen Flügeln versehen sein, die zunächst nach oben umgeklappt sind und ebenfalls klebende Abschnitte aufweisen. Im Rahmen einer solchen, in den Figuren nicht dargestellten Ausgestaltung ist dann eine separate Abdeckung der nach oben umgeklappten Flügel notwendig.

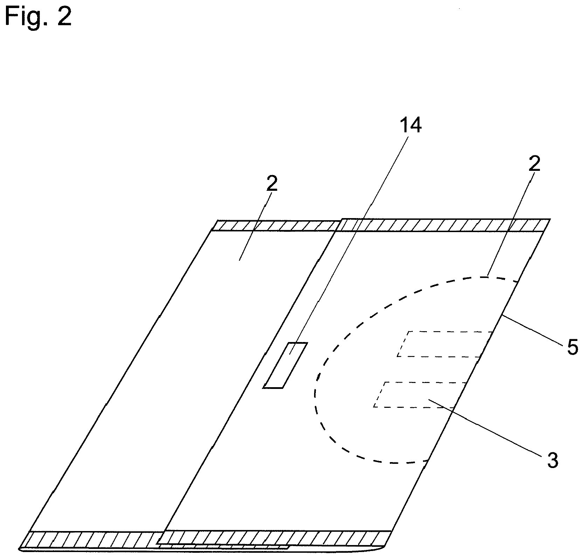

Die Fig. 2 zeigt die mit der Einschlagfolie 2 einzeln verpackte Damenbinde 1, wobei die zunächst gemäß der Fig. 1 flachliegende Anordnung bezogen auf die Längsrichtung L der Damenbinde 1 C-förmig zusammengefaltet ist, so dass dann in Längsrichtung L gesehen die Enden der Damenbinde 1 über einen Mittelabschnitt angeordnet sind. Aus der Fig. 2 ersichtliche Knicklinien 5 sind zur Verdeutlichung auch in der Fig. 1 eingezeichnet.

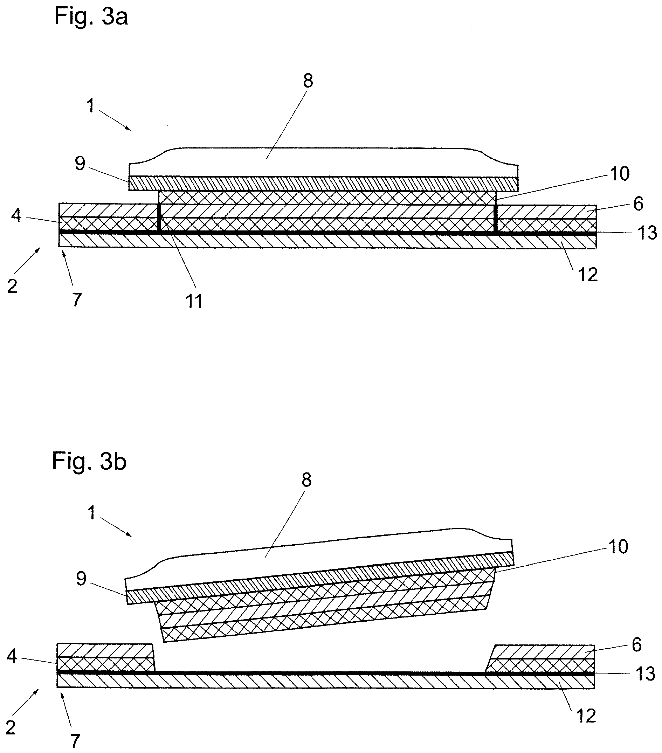

Gemäß der Fig. 3A, einem Schnitt entlang der Linie A-A der Fig. 1, ist die Einschlagfolie 2 mehrlagig ausgestaltet und weist eine erste Außenlage 6, eine zweite Außenlage 7 und zwischen der ersten Außenlage 6 und der zweiten Außenlage 7 eine Schicht des Haftklebstoffs 4 auf.

Die auf der Einschlagfolie 2 angeordnete Damenbinde 1 umfasst einen Grundkörper 8, der aus mehreren Schichten gebildet ist und neben einer im Einzelnen nicht dargestellten flüssigkeitsdurchlässigen Oberseite auch einen saugenden Kern umfasst. Des Weiteren umfasst die Damenbinde 1 eine Rückwand 9, welche vorzugsweise atmungsaktive Eigenschaften aufweist, jedoch wasserdicht ist.

Die Damenbinde 1 ist mit der Rückwand 9 durch einen nicht lösbaren Klebstoff 10 auf die erste Außenlage 6 aufgeklebt. Dort, wo der nicht lösbare Klebstoff 10 aufgebracht ist, wurde gemäß dem Ausführungsbeispiel der Fig. 3A zuvor durch Stanzen oder durch einen Laser in der ersten Außenlage 6 eine Schwächungslinie 11 erzeugt, welche im Rahmen der Erfindung jedoch optional ist. Alternativ kann die erste Außenlage auch so ausgestaltet sein, dass diese leicht bei einem Abziehen der Damenbinde 1 von der Einschlagfolie 2 aufbrechen kann.

Erfindungsgemäß ist die Verbundhaftung der Schicht aus Haftklebstoff 4 gegenüber der ersten Außenlage 6 größer als die Verbundhaftung zwischen der Schicht aus Haftklebstoff 4 und der zweiten Außenlage 7.

In dem dargestellten Ausführungsbeispiel ist die zweite Außenlage zweischichtig ausgeführt und umfasst eine Trägerschicht 12 und angrenzend an die Schicht aus Haftklebstoff 4 ein Release-Schicht 13, die gegenüber dem Haftklebstoff 4 eine besonders niedrige Verbundhaftung aufweist. In dem dargestellten Ausführungsbeispiel ist die erste Außenlage 6 von lediglich einer Folienschicht gebildet, wobei aber auch ohne Einschränkung eine mehrschichtige Ausgestaltung in Betracht kommt.

In dem dargestellten Ausführungsbeispiel liegt die Dicke der Trägerschicht 12 sowie der ersten Außenlage 6 in einem Bereich zwischen 5 µm und 20 µm, beispielsweise bei etwa 13 µm. Als Material ist Polyolefin, vorzugsweise Polyethylen (PE) vorgesehen, so dass im gefalteten Zustand gemäß der Fig. 2 die erste Außenlage 6 mit der Trägerschicht 12 der zweiten Außenlage 7 durch eine Heißsiegelung 14 verbunden werden kann, so dass ein separater Klebestreifen für einen Verschluss der Verpackung gemäß der Fig. 2 entfallen kann.

Wenn gemäß der Fig. 3B die Damenbinde 1 von der Einschlagfolie 2 abgezogen wird, verbleibt entlang des nicht lösbaren Klebstoffs 10 die erste Außenlage 6 mit der Schicht aus Haftklebstoff 4 an der Damenbinde 1, wodurch die in der Fig. 1 dargestellten Klebebereiche 3 erzeugt werden.

Die so gebildeten Klebebereiche 3 erstrecken sich vorzugsweise über zwischen 20 % bis 80 % einer Grundfläche der Damenbinde 1 in ihrem flachgelegten Zustand (siehe Fig. 1).

Die Einschlagfolie 2 kann sowohl vollständig durch Coextrusion oder auch durch eine Kaschierung gebildet werden. Beispielsweise können die erste Außenlage 6 und die zweite Außenlage 7 durch den Haftklebstoff 4 kaschiert werden, wenn dieser thermoplastisch ist und im schmelzflüssigen Zustand zwischen die erste Außenlage 6 und die zweite Außenlage 7 eingebracht wird. Der Haftklebstoff 4 dient dann bei der Kaschierung als Heißklebstoff (Hotmelt-Klebstoff).

Der Haftklebstoff 4 kann beispielsweise auf der Basis von Styrol-Block-Copolymer, insbesondere SIS oder auch auf Acrylat-Basis gebildet sein.

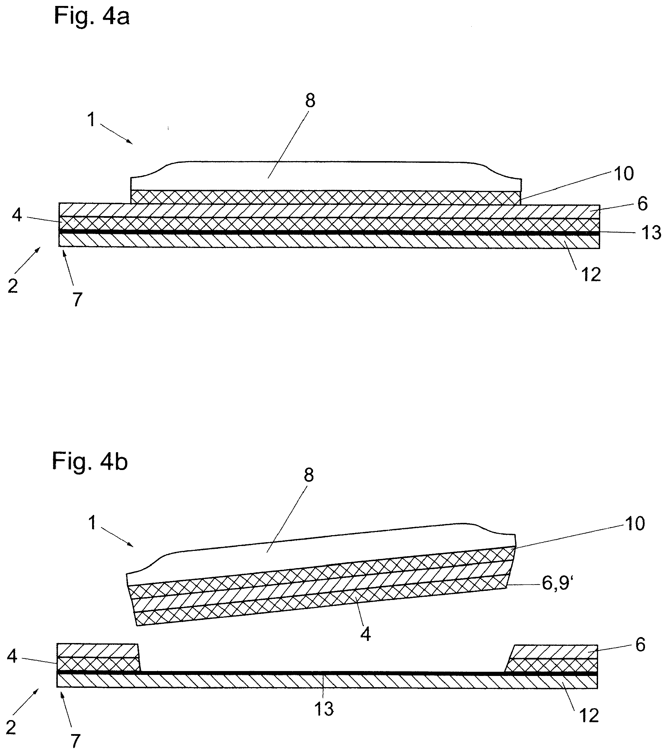

Die Fig. 4A und 4B zeigen eine alternative Ausgestaltung der Erfindung, wobei lediglich der Grundkörper 8 der Damenbinde 1 mit dem nicht lösbaren Klebstoff 10 auf die erste Außenlage 6 aufgeklebt wird.

Die Verklebung mit dem nicht lösbaren Klebstoff 10 erfolgt vorzugsweise an der gesamten Fläche des Grundkörpers 8, wobei dann nachfolgend die bei einem Abziehen der Damenbinde 1 von der Einschlagfolie 2 über den nicht lösbaren Klebstoff 10 auf dem Grundkörper 8 verbleibende erste Außenlage 6 eine Rückwand 9' der Damenbinde 1 bildet, wodurch eine weitere konstruktive Vereinfachung erreicht werden kann.

Um auch bei einer Ausgestaltung gemäß den Fig. 4A und 4B eine Atmungsaktivität zu erreichen, kann die erste Außenlage 6 bei ihrer Herstellung mit Füllstoffen, beispielsweise Kreide versehen werden, wobei dann durch eine Dehnung der ersten Außenlage 6 an den Partikeln des Füllstoffes atmungsaktive Poren gebildet werden.

Wenn die erste Außenlage 6 mit der zweiten Außenlage 7 kaschiert ist, ist es ausreichend, vor der Kaschierung lediglich die erste Außenlage 6 zu recken. Wenn dagegen die gesamte Einschlagfolie 2 durch Coextrusion hergestellt ist, ist auch die gesamte Coextrusionsfolie einem Reckprozess zu unterziehen, um in der beschriebenen Weise atmungsaktive Eigenschaften der ersten Außenlage 6 zu erzielen.

## Patentansprüche

1. Mehrlagige Einschlagfolie (2) für Hygieneprodukte, insbesondere Damenbinden (1), mit einer ersten Außenlage (6), einer zweiten Außenlage (7) und mit einer Haftklebstoffschicht zwischen der ersten Außenlage (6) und der zweiten Außenlage (7), wobei die Verbundhaftung zwischen der Haftklebstoffschicht und der ersten Außenlage (6) größer ist als die Verbundhaftung zwischen der Haftklebstoffschicht und der zweiten Außenlage (7) und wobei die Gesamtdicke der ersten und zweiten Außenlage (6, 7) mit der dazwischen angeordneten Haftklebstoffschicht zwischen 20 µm und 80 µm liegt.

2. Mehrlagige Einschlagfolie (2) nach Anspruch 1, wobei die zweite Außenlage (7) eine an die Haftklebstoffschicht angrenzende Release-Schicht (13) aufweist.

3. Mehrlagige Einschlagfolie (2) nach Anspruch 1 oder 2, wobei die erste Außenlage (6) und die zweite Außenlage (7) jeweils eine Dicke zwischen 5 µm bis 30 µm und die Haftklebstoffschicht eine Dicke zwischen 5 µm und 25 µm aufweisen.

4. Mehrlagige Einschlagfolie (2) nach einem der Ansprüche 1 bis 3, wobei die erste Außenlage (6), die zweite Außenlage (7) und die Haftklebstoffschicht gemeinsam coextrudiert sind.

5. Mehrlagige Einschlagfolie (2) nach einem der Ansprüche 1 bis 4, wobei die Haftklebstoffschicht auf der Basis von Styrol-Block-Copolymer, insbesondere Styrol-Isopren-Styrol-Block-Copolymer gebildet ist.

6. Mehrlagige Einschlagfolie (2) nach einem der Ansprüche 1 bis 3, wobei die Haftklebstoffschicht thermoplastisch ist und wobei die erste Außenlage (6) und die zweite Außenlage (7) getrennt gefertigt und durch die Haftklebstoffschicht als Heißklebstoff kaschiert sind.

7. Mehrlagige Einschlagfolie (2) nach Anspruch 6, wobei die Haftklebstoffschicht auf der Basis von Acrylat gebildet ist.

8. Mehrlagige Einschlagfolie (2) nach einem der Ansprüche 1 bis 7, wobei die erste Außenlage (6) und die zweite Außenlage (7) jeweils als polymeren Hauptbestandteil ein Polyolefin, insbesondere Polyethylen, aufweisen.

9. Mehrlagige Einschlagfolie (2) nach einem der Ansprüche 1 bis 8, wobei zumindest die erste Außenlage (6) gereckt ist.

10. Mehrlagige Einschlagfolie (2) nach Anspruch 9, wobei die erste Außenlage (6) Füllstoffe enthält und durch das Recken gebildete atmungsaktive Poren aufweist.

11. Einzeln eingeschlagene Damenbinde (1) mit einem sich über die Damenbinde (1) herauserstreckenden Zuschnitt der mehrlagigen Einschlagfolie (2) nach einem der Ansprüche 1 bis 10, wobei zumindest ein Grundkörper (8) der Damenbinde (1) nicht lösbar mit der ersten Außenlage (6) verklebt ist.

12. Einzeln eingeschlagene Damenbinde (1) nach Anspruch 11, wobei der Grundkörper (8) der Damenbinde (1) vollflächig mit der ersten Außenlage (6) verklebt ist, so dass die erste Außenlage (6) eine Rückwand (9) der Damenbinde (1) bildet.

13. Einzeln eingeschlagene Damenbinde (1) nach Anspruch 11, wobei der Grundkörper (8) der Damenbinde (1) mit einer Rückwand (9) nicht lösbar mit der ersten Außenlage (6) verklebt ist, wobei die nicht lösbar verklebte Fläche zwischen 20 % und 80 % einer Grundfläche der Damenbinde (1) im flachgelegten Zustand entspricht.

14. Einzeln eingeschlagene Damenbinde (1) nach Anspruch 13, wobei die Rückwand (9) und die erste Außenlage (6) entlang von zwei in einer Längsrichtung der Damenbinde (1) verlaufenden Streifen untrennbar verklebt sind.

15. Einzeln eingeschlagene Damenbinde (1) nach Anspruch 13, wobei die erste Außenlage (6) an den Rändern der verklebten Fläche eine Schwächungslinie (11) aufweist.
